# FASCICULE DE BREVET EUROPEEN

(11) **EP 2 723 385 B1**
(45) Date de publication et mention de la délivrance du brevet: **31.07.2019**
(21) Numéro de dépôt: 12731592.7
(22) Date de dépôt: 21.06.2012
(51) Int. Cl.: A61K 47/10, A61K 47/12, A61K 47/38, A61K 47/44, A61P 31/22, A61K 9/00, A61K 9/70, A61K 47/02

(54) **COMPOSITION FILMOGÈNE ET SON UTILISATION POUR LE TRAITEMENT DE L'HERPÈS**
FILMBILDENDE ZUSAMMENSETZUNG UND IHRE VERWENDUNG ZUR BEHANDLUNG VON HERPES
FILM-FORMING COMPOSITION, AND USE THEREOF FOR TREATING HERPES

(30) Priorité: 22.06.2011 FR 1155493
(43) Date de publication de la demande: 30.04.2014
(73) Titulaire: Urgo Recherche Innovation et Développement, 21300 Chenove (FR)
(72) Inventeur: DERAIN, Nathalie, F-21370 Prenois (FR)
(74) Mandataire: Cabinet Plasseraud
(86) Numéro de dépôt international: PCT/FR2012/051417
(87) Numéro de publication internationale: WO 2012/175879

(56) Documents cités:
- WO-A1-89/10745
- WO-A1-90/01046
- WO-A1-97/25018
- WO-A1-99/49835
- WO-A1-2005/070378
- WO-A2-2004/021968
- WO-A2-2007/147052
- WO-A2-2008/070602
- FR-A1- 2 783 832
- MD-F1- 1 779
- US-A1- 2010 297 043

## Description

La présente invention a pour objet une composition filmogène à base d'éthylcellulose permettant la formation d'un film protecteur sur la peau ou les muqueuses ainsi que son application dans le traitement des lésions cutanées, en particulier de l'herpès.

Le virus de l'herpès (Herpes Simplex Virus ou HSV) est présent dans une grande partie de la population. Bien que l'infection soit la plupart du temps asymptomatique, elle peut provoquer des lésions cutanées sous forme d'éruptions vésiculeuses, en particulier au niveau des muqueuses. Ces éruptions, qui commencent par une simple démangeaison et l'apparition de rougeurs, entraînent en quelques heures le développement de papules. Au cours de leur mûrissement, les papules deviennent des cloques puis des vésicules contenant un liquide clair. Les vésicules finissent par éclater, libérant ainsi le liquide. À ce stade, le risque de transmission ou de propagation du virus est particulièrement élevé. Les vésicules laissent alors place à une plaie qui forme une croûte avant de cicatriser. Ces derniers stades de développement de l'éruption s'avèrent, en outre, particulièrement douloureux.

Les éruptions herpétiques locales, communément appelées boutons de fièvre dans le cas d'herpès labial, sont le plus souvent traitées par l'application de compositions sous forme de pommade, d'onguent ou de gel comprenant des agents actifs. Cependant, du fait des mouvements et/ou des frottements intervenant sur les zones de traitements concernées, ces compositions ont tendance à ne pas rester localisées sur la plaie, limitant ainsi l'action des agents actifs. L'application de ces compositions sur la zone à traiter nécessite donc d'être souvent renouvelée.

En outre, ce type de formulation galénique ne permet pas de constituer une barrière efficace au virus herpétique, pourtant très contagieux. Des compositions de type patchs, plus occlusives, ont ainsi été proposées pour le traitement de l'herpès labial, mais ces patchs génèrent une sensation désagréable de corps étranger lorsqu'ils sont appliqués sur les zones à traiter.

D'autres solutions consistent en l'utilisation de compositions filmogènes. Ainsi, le document US 5,081,158 décrit une composition filmogène à base d'hydroxypropylcellulose (HPC) permettant de prolonger le maintien des agents actifs sur la zone à traiter.

Le document EP-A-679390 décrit, quant à lui, une formulation topique pulvérisable pour le traitement de la peau ou des muqueuses, et en particulier des lésions herpétiques, comprenant un agent actif dispersé dans une solution aqueuse de polymère cellulosique. Cette composition permet notamment d'éviter de toucher les lésions avec les doigts lors de l'application, ce qui peut s'avérer très douloureux.

Le document EP-A-289900 décrit que la pénétration cutanée d'un agent antibactérien peut être améliorée en administrant ledit agent dans une composition filmogène comprenant un polymère non soluble dans l'eau, une huile végétale et un solvant volatil.

Le document WO2007/147052 décrit des compositions topiques permettant de véhiculer et de libérer des actifs, comprenant, dans un solvant volatil, ledit actif en une concentration élevée associé à un inhibiteur de cristallisation permettant de retarder la cristallisation de l'agent actif très concentré.

Le document WO89/10745 décrit des formulations à base d'hydroxypropylcellulose permettant de former un film protecteur sur les muqueuses.

Cependant, les films formés par les compositions proposées dans ces documents présentent l'inconvénient de ne pas constituer une barrière efficace vis-à-vis de la propagation du virus de l'herpès.

Il existe donc un besoin pour des compositions filmogènes destinées au traitement de l'herpès, notamment de l'herpès labial, formant un film étanche au virus HSV et aux bactéries extérieures, de manière à prévenir toute contamination de la plaie herpétique à traiter, et toute diffusion du virus HSV à des sujets non porteurs du virus.

La société demanderesse a donc mis au point une composition filmogène permettant la formation d'un film constituant une barrière efficace vis-à-vis de la propagation du virus de l'herpès. De plus, la composition selon l'invention présente l'avantage d'être imperméable aux agents infectieux.

Le film ainsi obtenu permet, d'une part, de protéger la lésion contre l'intrusion de bactéries, prévenant ainsi le risque de surinfection, et d'autre part, d'éviter la propagation du virus de l'herpès responsable de la lésion à des sujets sains.

Ainsi, la présente invention concerne une composition topique filmogène comprenant, dans un milieu pharmaceutiquement acceptable :
- 8 à 30% en poids, de préférence de 8 à 20% en poids, d'éthylcellulose ;
- 0,1 % à 20%, de préférence de 2 à 12 % en poids, d'un agent auxiliaire de filmification ;
- 0,1 à 5% en poids, de préférence 0,5 à 2% en poids, d'un ou plusieurs triacides dont l'un au moins est l'acide borique; et
- 60 à 95% en poids, de préférence 65 à 85% en poids, d'un solvant organique les pourcentages étant exprimés en poids par rapport au poids total de la composition, ledit agent auxiliaire de filmification étant choisi parmi les huiles végétales.

Le film obtenu avec les compositions selon l'invention est souple, confortable et ne procure, en particulier, pas de gêne ou de sensation de corps étranger lorsqu'il est appliqué sur la zone à traiter.

Selon un mode préféré de réalisation, le film obtenu avec les compositions selon l'invention est incolore.

On entend par « composition topique » au sens de la présente demande, une forme d'administration d'une composition sur une région déterminée du corps.

Selon l'invention, la composition topique peut être une solution, un gel, une crème, une pommade, une lotion ou un vernis.

L'invention concerne également l'utilisation d'une telle composition pour le traitement de l'herpès, notamment labial.

### Ethylcellulose

La composition selon l'invention comprend au moins un polymère filmogène d'éthylcellulose. Il a, en effet, été observé que l'introduction d'éthylcellulose dans des compositions topiques pharmaceutiques ou dermatologiques permet d'améliorer l'adhérence, la résistance aux frottements, la viscosité et l'hydrophobicité de ces compositions.

L'éthylcellulose est un polymère filmogène.

On entend par "polymère filmogène", un polymère apte à former à lui seul ou en présence d'un agent auxiliaire de filmification, un film continu et adhérent sur un support, notamment sur la peau ou les muqueuses.

L'éthylcellulose est un éther éthylique de cellulose, et se présente sous la forme d'un polymère à longues chaînes constituées d'unités anhydroglucoses reliées entre elles par des groupements acétals.

Chaque unité anhydroglucose possède trois groupements hydroxyles substituables en tout ou partie, qui peuvent réagir selon le schéma réactionnel suivant:

RONₐ + C₂H₅ Cl → ROC₂H₅ + NₐCl

La substitution des trois groupements hydroxyles conférerait à chaque unité anhydroglucose un taux de substitution de 3 ou une teneur en éthoxyle de 54,88%.

Les polymères d'éthylcellulose utilisables dans la composition selon l'invention sont notamment ceux présentant un taux de substitution de leurs groupements hydroxyles compris entre 2,5 et 2,60 par unité d'anhydroglucose, ou dont la teneur en éthoxyle est comprise entre 44 et 50% par unité d'anhydroglucose.

A titre d'exemples non limitatifs de polymères d'éthylcellulose pouvant être utilisés dans le cadre de la présente demande, on peut citer ceux fabriqués par Dow Chemical Corporation (Midland, Michigan) et commercialisés sous la dénomination « ETHOCEL », ou ceux qui sont fabriqués par Hercules, Inc (Wilmington, Delaware).

La composition selon l'invention comprend de 8 à 30% en poids, de préférence de 8 à 20% en poids, et plus préférentiellement de 8 à 15% en poids, d'éthylcellulose, par rapport au poids total de la composition.

### Agent auxiliaire de filmification

Pour améliorer les propriétés filmogènes de la composition, un agent auxiliaire de filmification est ajouté.

L'agent auxiliaire de filmification est, bien évidemment, différent du solvant organique. L'agent auxiliaire de filmification est choisi parmi les huiles végétales.

Un tel agent auxiliaire de filmification peut être choisi parmi tous les composés connus de l'homme du métier comme étant susceptibles de remplir la fonction recherchée. Il est notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges. L'huile peut également être un dérivé d'une des huiles végétales citées précédemment. Il peut s'agir d'huile hydrogénée ou non, peroxydée ou non.
et leurs mélanges.

L'agent auxiliaire de filmification selon l'invention est choisi parmi les huiles végétales, telles que l'huile de ricin.

Selon un mode plus préféré de réalisation, l'agent auxiliaire de filmification est l'huile de ricin.

L'agent auxiliaire de filmification est présent dans la composition selon l'invention en une teneur allant de 0,1 % à 20%, et de préférence de 2 % à 12 % en poids, par rapport au poids total de la composition.

### Triacide

La composition selon l'invention comprend en outre un ou plusieurs triacides dont l'un au moins est l'acide borique.

Le triacide permet notamment d'améliorer la cohésion, et par conséquent les propriétés mécaniques du film.

De manière avantageuse, le triacide est choisi parmi un ou plusieurs triacides minéraux ou organiques, tels que l'acide phosphorique, l'acide borique, l'acide citrique ou leurs mélanges.

En particulier, la composition selon l'invention comprend de préférence un mélange d'acide borique et d'acide citrique, de préférence en un rapport pondéral compris entre 1:2 et 2:1, de préférence 1:1.

Le triacide est présent dans la composition selon l'invention en une teneur allant de 0,1 à 5% en poids, de préférence 0,5 à 2% en poids, par rapport au poids total de la composition.

L'association dans les compositions selon l'invention d'un polymère d'éthylcellulose, d'un solvant organique et d'au moins un triacide, confère au film obtenu des propriétés particulièrement inattendues, telles que notamment une excellente étanchéité du film aux virus et aux bactéries, et une intégrité de la structure du film pendant toute l'application.

### Solvant organique

La composition selon l'invention comprend au moins un solvant organique.

Le solvant organique peut notamment être choisi parmi :
- les cétones telles que la méthyléthylcétone, la méthylisobutylcétone, la diisobutylcétone, l'isophorone, la cyclohexanone, l'acétone;
- les alcools liquides à température ambiante tels que l'éthanol, l'isopropanol, le diacétone alcool, le 2-butoxyéthanol, le cyclohexanol, le n-propanol, le n-butanol ; les éthers de propylène glycol liquides à température ambiante tels que le monométhyléther de propylène glycol, l'acétate de monométhyl éther de propylène glycol, le mono n-butyl éther de dipropylène glycol ;
- les glycols tels que l'éthylène glycol, le propylène glycol, le pentylène glycol, le glycérol
- les éthers cycliques tels que la γ-butyrolactone ;
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle ;
- les éthers tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tels que le carbonate de propylène, le carbonate de diméthyle,
- les acétals tels que le méthylal ;
et leurs mélanges.

Selon un mode préféré de réalisation, le solvant organique est volatil.

Par " solvant organique volatil", on entend un solvant organique susceptible de s'évaporer au contact de la peau en moins d'une heure, à température ambiante et pression atmosphérique. Le solvant organique volatil est liquide à température ambiante, présente notamment une pression de vapeur non nulle à température ambiante et pression atmosphérique, en particulier il présente une pression de vapeur allant de 0,13 Pa à 40 000 Pa (10<-3> à 300 mm de Hg), et de préférence allant de 1,3 Pa à 8000 Pa (0,01 à 60 mm de Hg).

Selon un mode particulièrement préféré de réalisation, le solvant organique est choisi parmi les alcools liquides à température ambiante tels que l'éthanol, l'isopropanol, le diacétone alcool, le 2-butoxyéthanol, le cyclohexanol et leurs mélanges, et de préférence l'éthanol.

Selon un mode plus préféré de réalisation, le solvant organique utilisé dans les compositions selon l'invention est l'éthanol.

Le solvant organique peut représenter de 60 à 95% en poids, de préférence de 65 à 85% en poids, et de préférence de 65 à 80% en poids, par rapport au poids total de la composition.

### Milieu pharmaceutiquement acceptable

La composition selon l'invention comprend un milieu pharmaceutiquement acceptable pouvant contenir de l'eau.

Par milieu pharmaceutiquement acceptable, on entend, au sens de la présente demande, un milieu compatible avec la peau.

L'eau peut notamment être présente dans la composition selon l'invention en une teneur allant de 1 à 20% en poids, de préférence de 5 à 15% en poids.

En particulier, la composition comprend un mélange d'eau (i.e. à titre de milieu pharmaceutiquement acceptable) et d'éthanol (i.e. à titre de solvant organique) en un rapport pondéral compris entre 1:2 et 1:10, de préférence entre 1:4 et 1:8, et de préférence encore entre 1:5 et 1:6 en poids.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les conservateurs, les agents dépigmentants, les agents hydratants, les filtres solaires, les agents restructurants.

### Agents actifs

Dans un mode particulier de réalisation, la composition selon la présente invention ne contient pas d'agent pharmaceutiquement actif.

En effet, les inventeurs ont observé, de manière surprenante, qu'un film formé par application d'une composition selon l'invention sur une zone de peau ou de muqueuse présentant une éruption herpétique permettait le traitement de celle-ci, et ce même et surtout lorsque la composition selon l'invention ne contient pas d'agent pharmaceutiquement actif.

### Utilisation de la composition

Selon un mode particulier de réalisation, l'invention a pour objet la composition telle que définie précédemment pour son utilisation dans une méthode de traitement de l'herpès, en particulier labial.

L'invention a également pour objet, selon un autre mode de réalisation, la composition telle que définie précédemment pour son utilisation dans une méthode de cicatrisation des plaies générées par le virus de l'herpès, sur la peau ou les muqueuses.

En appliquant la composition selon l'invention sur une éruption herpétique dès les premiers signes annonciateurs de la lésion (démangeaisons, picotements, rougeurs), il est possible de limiter la formation de papules. Lorsqu'elle est appliquée après la formation des papules, la composition selon l'invention contribue à limiter la formation de vésicules et de croûtes.

Enfin, le film obtenu avec les compositions de l'invention permet également de favoriser la cicatrisation de l'éruption herpétique.

La présente invention est illustrée plus en détails dans les exemples non limitatifs décrits ci-après.

### Exemples

### Exemple 1 : Préparation de la composition A

Une composition A selon l'invention a été préparée de la façon suivante :

| Ingrédient | % massique |
|---|---|
| Ethylcellulose | 10 |
| Huile de ricin | 10 |
| Acide borique | 0,5 |
| Acide citrique | 0,5 |
| Ethanol | 67 |
| Eau déminéralisée | 12 |

L'huile de ricin, l'acide borique et l'acide citrique sont ajoutés dans le mélange eau/éthanol sous agitation maintenue pendant 10 min. L'éthylcellulose est ensuite dispersée dans le mélange. La composition A est récupérée après agitation du mélange pendant 30 min.

### Exemple 2 : Evaluation de l'imperméabilité du film obtenu avec la composition A aux agents infectieux

### 1. Matériel :

Les cellules utilisées sont des cellules Vero provenant de l'ATCC CCL-81 ou du laboratoire médical de Virologie de l'Institut Pasteur (Yasumunra Y. et al., 1962). Ces cellules dérivent d'un rein de singe vert d'Afrique.

Le milieu de culture et de titrage est un milieu DMEM (« Dulbecco/Vogt modifiedEagle's Minimal Essential Médium » en terminologie anglosaxone) supplémenté avec 2% de glutamine, 4% SVF et 1% de sulfate de gentamicine.

Le milieu de dilution est un milieu DMEM supplémenté avec 2% de glutamine et 1% de sulfate de gentamicine.

Le virus utilisé est le **Pseudo Rabies Virus** (PRV) (Virus de la maladie d'Aujesky) :ATCC VR-135 (Aujesky A., 1902; Roizrnan R. et al., 1981).

Le PRV appartient à la famille des *Herpesviridae*, sous-famille des *Aalphaherpèsviridae,* genre Varicellovirus.

Ce virus est utilisé comme modèle de l'herpès humain de type 1.

Les plaques de culture et les cupules utilisées proviennent du fournisseur Dutscher. Les références de ces plaques et cupules sont les suivantes :
- Multiwell 6 well : ref : 3502.
- Filtre : ref : 3091.

### 2. Evaluation de l'étanchéité et de l'intégrité du film obtenu avec la composition A

Préalablement à l'évaluation de l'imperméabilité du film formé au virus de l'herpès, on a démontré l'étanchéité du film au milieu de culture et son intégrité lors d'un contact prolongé avec ledit milieu de culture.

La composition A est introduite dans 1 puits de plaque 6 puits. La quantité versée (0,5 à 1 cm) doit être suffisante pour pouvoir tremper la cupule sur une hauteur d'environ 3 mm. A l'aide d'une pince, la cupule est trempée dans la composition en la maintenant en biais afin d'éviter la formation de bulles. Une fois la surface du fond de la cupule totalement en contact avec la composition, la cupule est retirée délicatement.

Le fond de la cupule est raclé à l'aide d'une spatule afin d'enlever le surplus de produit. Puis, elle est déposée à l'envers afin d'assécher le film.

L'épaisseur du film ainsi formé est de l'ordre de 30 µm. 8 cupules « filmées » sont ainsi réalisées.

### a. Test d'étanchéité du film obtenu avec la composition A:

Deux cupules « filmées » et une cupule témoin sans film ont été introduites dans les puits d'une plaque de culture contenant du sérum physiologique (NaCl 9g/L).

Dans chacune des cupules, le milieu de culture DMEM a été introduit. Le milieu de culture DMEM utilisé pour le présent test est de couleur rose.

Après 24h à 37°C, on a observé que le sérum physiologique du puits contenant la cupule témoin était devenu rose. Le DMEM de la cupule témoin a donc migré vers le milieu de sérum physiologique.

Le sérum physiologique du puits contenant la cupule « filmée » est quant à lui resté incolore : le DMEM n'a pas migré.

Ces résultats montrent que les films formés avec les compositions selon la présente invention sont étanches au milieu de culture.

### b. Test d'intégrité du film obtenu avec la composition A :

On a mis en contact le film obtenu avec la composition A pendant 24h à 37°C avec le milieu de culture DMEM.

Après 24h, le film reste intègre, et s'opacifie juste légèrement.

### c. Test de l'effet barrière du film selon l'invention au virus de l'herpès

### i. Méthode

Dans 16 puits de plaques 6 puits, 3 ml de milieu de culture DMEM ont été introduits.

Huit cupules « filmées » et huit cupules non filmées (contrôle positif) sont placées dans les puits.

200µL de suspension virale de PRV sont ajoutés dans chaque cupule.

Les plaques et cupules sont incubées à 37°C ± 2°C en présence de 5 ± 0.5% CO₂.

Après 1, 2, 4 et 6 heures d'incubation, deux cupules « filmées » et deux cupules non filmées par heure sont retirées des plaques afin d'établir une cinétique.

Chaque surnageant est récolté individuellement pour être titré.

### ii. Protocole de titrage :

Chaque surnageant collecté dans les puits contenant les cupules « filmées » est dilué à la dilution non toxique et non virucide du produit testé, c'est-à-dire au 1/10 et au 1/100 dans du milieu de dilution, et est titré sur 2 plaques 96 puits comme décrit ci-après.

Soit un total de 32 plaques 96 puits.

### • Procédure

Pour chaque plaque 96 puits, 50 µl de milieu de dilution sont ajoutés dans chaque puits. Ensuite, 25 µl de chaque surnageant dilué ou de contrôle positif sont ajoutés et homogénéisés dans les huit puits de la première colonne de la première plaque.

25 µl sont ensuite transférés de la colonne 1 à la colonne 2 (dilution au 1/3) et la solution est homogénéisée. Cette étape est répétée de la colonne 2 jusqu'à la colonne 11.

25 µl sont ensuite transférés de la colonne 11 (première plaque) à la colonne 1 (deuxième plaque). Cette étape est répétée de la colonne 2 jusqu'à la colonne 11.

Les colonnes 12 servent de témoin cellulaire. 50 µl d'une suspension de cellules Véro à 2.5^{∗}10⁵ cellules/ml sont ajoutés dans tous les puits.

Les plaques sont ensuite incubées entre 6 et 9 jours à 37°C ± 2°C en présence de 5% ± 0.5% de C02.

### • Témoin de titrage

Le stock viral PRV est titré sur 1 plaque 96 puits en parallèle.

### iii. Résultats

Les résultats des titrages des contrôles positifs et des essais sont présentés dans le tableau 1 ci-dessous.

**Tableau 1 : Résultats des titrages**

| | **Temps d'incubation** | **Observation de l'effet cytopathique (dilution à 10⁻¹)** | **Titre en TCID₅₀/mL** | **Observation de l'effet cytopathique (dilution à 10⁻²)** | **Titre en TCID₅₀/mL** |
|---|---|---|---|---|---|
| **Contrôles positifs** | 1 h | oui | 4,43 10⁷ (7,65) | | |
| | 2 h | oui | 1,74 10⁷ (7,24) | | |
| | 4 h | oui | 2,63 10⁷ (7,42) | | |
| | 6 h | oui | 1,7 10⁸ (8,23) | | |
| **Film obtenu avec la composition A** | 1 h | non | <3,26 10³ (<3,51) | non | <3,26 10⁴ (<4,51) |
| | 2 h | non | <3,26 103 (<3,51) | non | <3,26 10⁴ (<4,51) |
| | 4 h | non | <3,26 103 (<3,51) | non | <3,26 10⁴ (<4,51) |
| | 6 h | non | <3,26 103 (<3,51) | non | <3,26 10⁴ (<4,51) |

Contrairement aux contrôles positifs, aucun effet cytopathique n'est observé pour les essais mettant en oeuvre une cupule « filmée » avec la composition A selon l'invention.

Ces résultats confirment donc l'imperméabilité du film formé par la composition selon l'invention aux virus PRV, modèle de l'herpès humain de type 1, et la constance de cette imperméabilité pendant toute la durée de l'application.

### Exemple 3 : Test comparatif de la résistance à la rupture de films formés à partir d'une composition selon l'invention (composition A de l'exemple 1) et de compositions comparatives ne comprenant pas d'acide borique (compositions B et C)

La composition B (sans acide borique) comprend:

| Ingrédient | % massique |
|---|---|
| Ethylcellulose | 10 |
| Huile de ricin | 10 |
| Acide citrique | 0,5 |
| Ethanol | 67,5 |
| Eau déminéralisée | 12 |

L'huile de ricin et l'acide citrique sont ajoutés dans le mélange eau/éthanol sous agitation maintenue pendant 10 min. L'éthylcellulose est ensuite dispersée dans le mélange. La composition B est récupérée après agitation du mélange pendant 30 min.

Une composition C (sans acides borique et citrique) a également été préparée :

| Ingrédient | % massique |
|---|---|
| Ethylcellulose | 10 |
| Huile de ricin | 10 |
| Ethanol | 68 |
| Eau déminéralisée | 12 |

L'huile de ricin est ajoutée dans le mélange eau/éthanol sous agitation maintenue pendant 10 min. L'éthylcellulose est ensuite dispersée dans le mélange. La composition C est récupérée après agitation du mélange pendant 30 min.

### Mesure de la résistance à la rupture des films:

Les compositions A, B et C sont enduites sur du polyester siliconé et séchées à l'air libre. Ces enductions sont effectuées à l'aide d'une table d'enduction permettant de régler l'épaisseur de film voulue. Dans le cas présent, le film présente une épaisseur de 50 µm

Une bande rectangulaire de film de 15 mm de large et de 150 mm de long est découpée à l'emporte-pièce. Cette bande rectangulaire est ensuite placée entre les mors d'un dynamomètre MTS (modèle DY30 avec un capteur de force de 10 Newtons) distants de 100 mm. Un essai de traction jusqu'à la rupture de l'éprouvette à la vitesse de 300 mm/min est alors réalisé.

La déformation est calculée par le logiciel Testwork en prenant le point maximal de la courbe (rupture). Le logiciel donne ainsi la force nécessaire à la rupture. Les résultats ont été reportés dans le tableau 2 ci-après.

**Tableau 2 : Résultats des mesures de résistance à la rupture**

| Caractéristiques | Composition A | Composition B | Composition C |
|---|---|---|---|
| Force de rupture Sens longitudinal (N/cm) | 1,6 | 1,4 | 1,2 |

La composition filmogène selon l'invention nécessite une force plus importante que les autres pour se déformer, ce qui confère au film une meilleure intégrité pendant toute l'application.

## Revendications

1. Composition topique filmogène comprenant, dans un milieu pharmaceutiquement acceptable:
- 8 à 30% en poids d'éthylcellulose ;
- 0,1 à 20% en poids d'un agent auxiliaire de filmification ;
- 0,1 à 5% en poids d'un ou plusieurs triacides dont l'un au moins est l'acide borique; et
- 60 à 95% en poids d'un solvant organique,
les pourcentages étant exprimés en poids, par rapport au poids total de la composition, ledit agent auxiliaire de filmification étant choisi parmi les huiles végétales.

2. Composition selon la revendication 1, **caractérisée en ce que** l'éthylcellulose est présente en une teneur allant de 8 à 20% en poids, et de préférence de 8 à 15% en poids, par rapport au poids total de la composition.

3. Composition selon l'une des revendications 1 ou 2, **caractérisée en ce que** les huiles végétales sont choisies parmi l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges, de préférence l'huile de ricin.

4. Composition selon l'une quelconque des revendications précédentes, **caractérisée en ce que** l'agent auxiliaire de filmification est présent en une teneur allant 2 à 12% en poids, par rapport au poids total de la composition.

5. Composition selon l'une quelconque des revendications précédentes, **caractérisée en ce que** le triacide comprend un mélange d'acide borique et d'acide citrique, de préférence en un rapport pondéral compris entre 1:2 et 2:1.

6. Composition selon l'une quelconque des revendications précédentes, **caractérisée en ce que** le solvant organique est choisi parmi les alcools liquides à température ambiante tels que l'éthanol, l'isopropanol, le diacétone alcool, le 2-butoxyéthanol, le cyclohexanol et leurs mélanges, et de préférence l'éthanol.

7. Composition selon l'une quelconque des revendications précédentes, **caractérisée en ce que** le solvant organique représente de 65 à 85% en poids, par rapport au poids total de la composition.

8. Composition selon l'une quelconque des revendications précédentes, **caractérisée en ce qu'**elle comprend de l'eau, de préférence en une teneur allant de 1 à 20% en poids, de préférence de 5 à 15% en poids.

9. Composition selon l'une quelconque des revendications précédentes, **caractérisée en ce qu'**elle ne contient pas d'agent pharmaceutiquement actif pour le traitement de l'herpès.

10. Composition selon l'une quelconque des revendications 1 à 6, pour son utilisation dans une méthode de traitement de l'herpès, en particulier de l'herpès labial.

## Patentansprüche

1. Topische filmbildende Zusammensetzung, welche in einem pharmazeutisch akzeptablen Medium umfasst:
- 8 bis 30 Gew.-% Ethylcellulose;
- 0,1 bis 20 Gew.-% eines filmbildenden Hilfsstoffs;
- 0,1 bis 5 Gew.-% einer oder mehrerer Trisäuren, von denen wenigstens eine Borsäure ist; und
- 60 bis 95 Gew.-% eines organischen Lösungsmittels,
wobei die Prozentsätze in Gewichtsprozenten ausgedrückt werden, bezogen auf das Gesamtgewicht der Zusammensetzung, wobei der filmbildende Hilfsstoff aus Pflanzenölen gewählt ist.

2. Zusammensetzung nach Anspruch 1, **dadurch gekennzeichnet, dass** die Ethylcellulose mit einem Gehalt von 8 bis 20 Gew.-% und vorzugsweise 8 bis 15 Gew.-%, bezogen auf das Gesamtgewicht der Zusammensetzung, vorliegt.

3. Zusammensetzung nach einem der Ansprüche 1 oder 2, **dadurch gekennzeichnet, dass** die Pflanzenöle gewählt sind aus Sesamöl, Rizinusöl, Mandelöl, Canolaöl, Haselnussöl, Pistazienöl, Leinöl, Borretschöl, Hanföl, Jojobaöl, Sonnenblumenöl, Weizenkeimöl, Mais- und/oder Maiskeimöl, Erdnussöl, Avocadoöl, Distelöl, Rapsöl, Olivenöl, Arganöl, Sonnenblumenöl, Traubenkernöl, Sojaöl, Nussöl, Kürbiskernöl, Palmöl, Kokosöl und Mischungen daraus, vorzugsweise Rizinusöl.

4. Zusammensetzung nach einem der vorhergehenden Ansprüche, **dadurch gekennzeichnet, dass** der filmbildende Hilfsstoff mit einem Gehalt von 2 bis 12 Gew.-%, bezogen auf das Gesamtgewicht der Zusammensetzung, vorliegt.

5. Zusammensetzung nach einem der vorhergehenden Ansprüche, **dadurch gekennzeichnet, dass** die Trisäure eine Mischung aus Borsäure und Zitronensäure umfasst, vorzugsweise mit einem Gewichtsverhältnis zwischen 1:2 und 2:1.

6. Zusammensetzung nach einem der vorhergehenden Ansprüche, **dadurch gekennzeichnet, dass** das organische Lösungsmittel ausgewählt ist aus bei Raumtemperatur flüssigen Alkoholen wie Ethanol, Isopropanol, Diacetonalkohol, 2-Butoxyethanol, Cyclohexanol und Mischungen daraus und vorzugsweise Ethanol.

7. Zusammensetzung nach einem der vorhergehenden Ansprüche, **dadurch gekennzeichnet, dass** das organische Lösungsmittel 65 bis 85 Gew.-%, bezogen auf das Gesamtgewicht der Zusammensetzung, ausmacht.

8. Zusammensetzung nach einem der vorhergehenden Ansprüche, **dadurch gekennzeichnet, dass** sie Wasser umfasst, vorzugsweise mit einem Gehalt von 1 bis 20 Gew.-%, vorzugsweise 5 bis 15 Gew.-%.

9. Zusammensetzung nach einem der vorhergehenden Ansprüche, **dadurch gekennzeichnet, dass** sie keinen pharmazeutischen Wirkstoff zur Behandlung von Herpes enthält.

10. Zusammensetzung nach einem der Ansprüche 1 bis 6 zur Anwendung bei einer Methode zur Behandlung von Herpes, insbesondere Lippenherpes.

## Claims

1. A topical film-forming composition comprising, in a pharmaceutically acceptable medium:
- 8% to 30% by weight of ethylcellulose;
- 0.1% to 20% by weight of an auxiliary film-forming agent;
- 0.1% to 5% by weight of one or more triacids, at least one of which is boric acid; and
- 60% to 95% by weight of an organic solvent,
the percentages being expressed by weight,
relative to the total weight of the composition said auxiliary film-forming agent being chosen from vegetable oils.

2. The composition as claimed in claim 1, **characterized in that** the ethylcellulose is present in a content ranging from 8% to 20% by weight, and preferably from 8% to 15% by weight, relative to the total weight of the composition.

3. The composition as claimed in either of claims 1 and 2, **characterized in that** the vegetable oils are chosen from sesame oil, castor oil, almond oil, canola oil, hazelnut oil, pistachio oil, linseed oil, borage oil, flaxseed oil, jojoba oil, sunflower oil, wheat germ oil, corn and/or corn germ oil, groundnut oil, avocado oil, safflower oil, rapeseed oil, olive oil, argan oil, sunflower oil, grapeseed oil, soybean oil, walnut oil, marrow seed oil, palm oil, coconut oil, and mixtures thereof or a derivative of one of the vegetable oils mentioned above or a hydrogenated or nonhydrogenated, and peroxidized or nonperoxidized oil and mixtures thereof, preferably castor oil.

4. The composition as claimed in any one of the preceding claims, **characterized in that** the auxiliary film-forming agent is present in a content ranging from 2% to 12% by weight, relative to the total weight of the composition.

5. The composition as claimed in any one of the preceding claims, **characterized in that** the triacid comprises a mixture of boric acid and citric acid, preferably in a weight ratio of between 1:2 and 2:1.

6. The composition as claimed in any one of the preceding claims, **characterized in that** the organic solvent is chosen from alcohols which are liquid at ambient temperature, such as ethanol, isopropanol, diacetone alcohol, 2-butoxyethanol, cyclohexanol, and mixtures thereof, and preferably ethanol.

7. The composition as claimed in any one of the preceding claims, **characterized in that** the organic solvent represents 65% to 85% by weight, relative to the total weight of the composition.

8. The composition as claimed in any one of the preceding claims, **characterized in that** it comprises water, preferably in a content ranging from 1% to 20% by weight and preferably from 5% to 15% by weight.

9. The composition as claimed in any one of the preceding claims, **characterized in that** it does not contain any pharmaceutically active agent for the treatment of herpes.

10. The composition as claimed in any one of claims 1 to 6, for use thereof in a method for treating herpes, in particular herpes labialis.
